# EUROPEAN PATENT APPLICATION

(11) **EP 3 932 288 A1**
(43) Date of publication of application: **05.01.2022**
(21) Application number: 21180548.6
(22) Date of filing: 21.06.2021
(51) Int. Cl.: A61B 1/00, A61B 1/05, G02B 23/24, G02B 7/02

(54) **ENDOSCOPE**

(30) Priority: 29.06.2020 JP 2020111758; 11.12.2020 JP 2020205827
(71) Applicant: Panasonic i-PRO Sensing Solutions Co., Ltd., Fukuoka City 812-8531 (JP)
(72) Inventor: KOHNO, Haruhiko, Fukuoka-shi, Fukuoka, 812-8531 (JP)
(74) Representative: Eisenführ Speiser

(57) **Abstract**

An endoscope includes two or more imaging modules having a lens barrel containing an optical system, an image sensor, and a sensor holding member that relatively fixes the lens barrel and the image sensor, a sub-frame that relatively fixes each of the two or more imaging modules, and an outer shell portion that accommodates and fixes the sub-frame and the two or more imaging modules.

## Description

### TECHNICAL FIELD

The present disclosure relates to an endoscope.

### BACKGROUND ART

Patent Literature 1 discloses an endoscope capable of increasing bonding strength of a cover lens in an endoscope tip structure where the cover lens is located on a tip slope surface formed in a tip body of an insertion portion of an endoscope and a prism and a rear group lens barrel are located behind the cover lens. In the endoscope, a counterbore accommodation recess is formed on the tip slope surface of the tip body and a lens accommodation recess is formed on a front surface of a prism receiving plate, and further the cover lens is accommodated and glued to the lens accommodation recess of the prism receiving plate. Also, a prism is adhesively fixed to a back surface of the prism receiving plate and adhesive agent is filled around the cover lens on a front surface of the prism receiving plate accommodated in the counterbore accommodation recess of the tip body, and then the prism receiving plate and cover lens are adhesively fixed to the counterbore accommodation recess.

### CITATION LIST

### PATENT LITERATURE

Patent Literature 1: JP-A-2011-218033

### SUMMARY OF INVENTION

The endoscope of Patent Literature 1 does not suggest a working environment (particularly atmosphere) at the time of assembling and fixing each part which is an element forming the endoscope. Since an endoscope is inserted into a patient's body, especially when used for medical purposes, a part in contact with the patient's body needs to be manufactured in a clean working environment. However, since the endoscope also has a function as a camera device, in particular, when an optical system or an image sensor which requires a precise adjustment jig or the like is assembled in the above-described extremely clean working environment, construction costs related to a manufacturing process of the endoscope will increase significantly.

The present disclosure is devised in view of the above-described circumstances of the related art and an object of the present disclosure is to provide an endoscope capable of separating an assembly process which requires cleanliness and an assembly process which does not require cleanliness and suppressing an increase in manufacturing costs.

The present disclosure provides an endoscope including two or more imaging modules having a lens barrel containing an optical system, an image sensor, and a sensor holding member that relatively fixes the lens barrel and the image sensor; a sub-frame that relatively fixes each of the two or more imaging modules; and an outer shell portion that accommodates and fixes the sub-frame and the two or more imaging modules.

According to the present disclosure, it is possible to separate an assembly process which requires cleanliness from an assembly process which does not require cleanliness and it is possible to suppress an increase in manufacturing costs.

### BRIEF DESCRIPTION OF DRAWINGS

Fig. 1 is a perspective view illustrating an external example of an endoscope system according to a first embodiment.
Fig. 2 is a perspective view of a rigid portion illustrated in Fig. 1.
Fig. 3 is a perspective view as seen through an inside of the rigid portion illustrated in Fig. 2.
Fig. 4 is an explanatory drawing illustrating an example of an assembling procedure of a 3D camera module.
Fig. 5 is a side cross-sectional view of the rigid portion illustrated in Fig. 2.
Fig. 6 is an exploded perspective view of an endoscope tip component and an imaging module illustrated in Fig. 3.
Fig. 7 is a flowchart illustrating an example of a procedure of a method for manufacturing an oblique-viewing endoscope according to the first embodiment.
Fig. 8 is a flowchart illustrating an example of a procedure for adjusting a 3D sub-frame.
Fig. 9 is a schematic view illustrating an example of a procedure of optical axis parallelism adjustment and image horizontal adjustment of the 3D sub-frame.
Fig. 10 is a perspective view of the endoscope tip component to which a cover glass is adhesively fixed.
Fig. 11 is a perspective view of the 3D sub-frame to which the endoscope tip component is adhesively fixed.
Fig. 12 is a perspective view as seen through a part of the rigid portion where an outer shell is formed by the endoscope tip component and a sheath.

### DESCRIPTION OF EMBODIMENTS

Hereinafter, embodiments in which an endoscope module, an endoscope, and a method for manufacturing an endoscope according to the present disclosure are specifically disclosed will be described in detail with reference to the drawings as appropriate. However, more detailed explanation than necessary may be omitted. For example, detailed explanations of already well-known matters and duplicate explanations for substantially the same configuration may be omitted. This is to avoid unnecessary redundancy of the following description and to facilitate the understanding of those skilled in the art. The accompanying drawings and the following description are provided for those skilled in the art to fully understand the present disclosure and are not intended to limit the subject matter described in the claims.

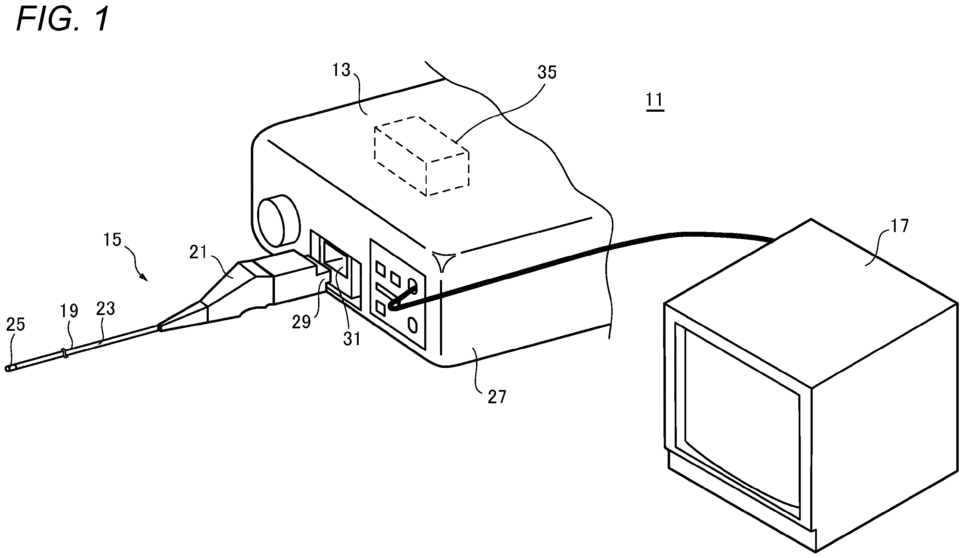

Fig. 1 is a perspective view illustrating an external example of an endoscope system 11 according to a first embodiment. As terms used here, upward and downward directions of a housing of a video processor 13 placed on a horizontal surface or a mounting surface such as a horizontal table are respectively referred to as "upper" and "lower". Further, a side where an endoscope captures an observation target is referred to as a "front (tip)" and a side connected to the video processor 13 is referred to as "rear".

The endoscope system 11 includes an oblique-viewing endoscope 15 as an example of an endoscope, the video processor 13, and a monitor 17. The oblique-viewing endoscope 15 used in the endoscope system 11 is an example, and as the endoscope forming the endoscope system 11, a direct-viewing endoscope (sometimes referred to as a "direct endoscope") may be used in addition to the oblique-viewing endoscope 15. The oblique-viewing endoscope 15 is, for example, a rigid mirror or a flexible mirror for medical use. The video processor 13 performs various image processes on a captured image (for example, including still images and videos) obtained by capturing an observation target (for example, an affected area) in a subject into which the oblique-viewing endoscope 15 is inserted and outputs the image. The monitor 17 displays an image according to a display signal output from the video processor 13. Various image processes include, but are not limited to, for example, color correction, gradation correction, and gain adjustment.

The oblique-viewing endoscope 15 includes a scope 19 inserted inside an observation target and a plug portion 21 to which a rear end portion of the scope 19 is connected. The scope 19 includes a soft portion 23 having a relatively long length and flexibility, and a rigid portion 25 having rigidity and provided at a tip of the soft portion 23. The oblique-viewing endoscope 15 can handle an area between the rigid portion 25 and the plug portion 21 as one finished product.

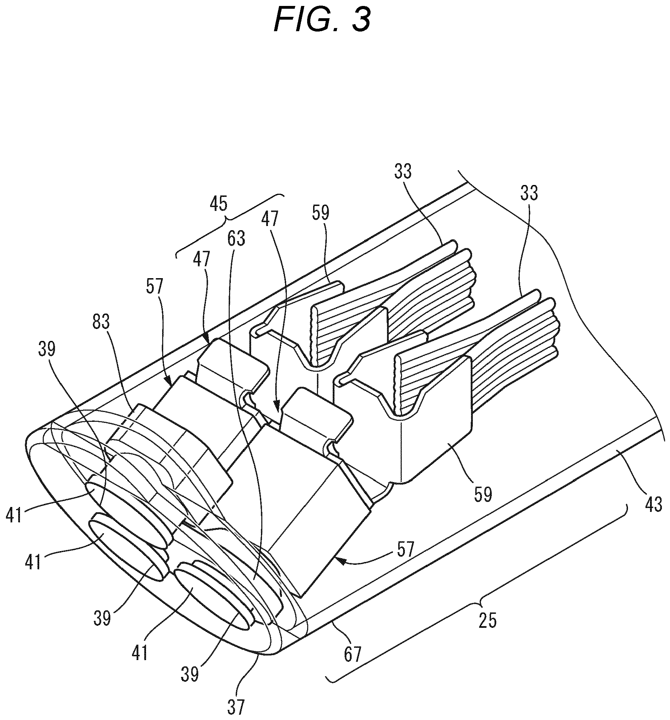

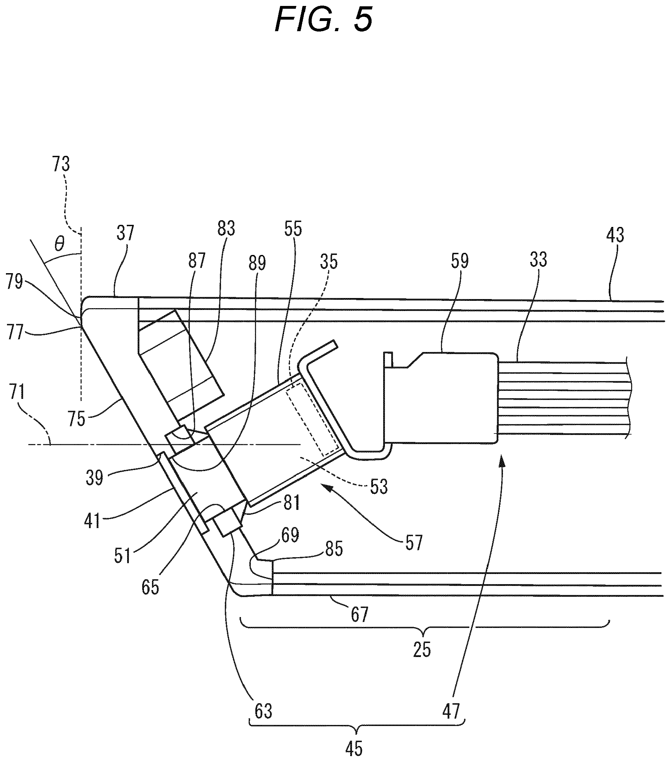

The video processor 13 has a housing 27, performs various image processes on the captured image captured by the oblique-viewing endoscope 15, and outputs a display signal after the image processes. On a front surface of the housing 27, a socket portion 31 into which a base end portion 29 of the plug portion 21 is inserted is disposed. The plug portion 21 is inserted into the socket portion 31 and the oblique-viewing endoscope 15 and the video processor 13 are electrically connected to each other, in such a manner that electric power and various signals (for example, captured image signals or control signals) can be transmitted and received between the oblique-viewing endoscope 15 and the video processor 13. The electric power and various signals are transmitted from the plug portion 21 to the soft portion 23 via a transmission cable 33 (see Fig. 3) inserted inside the scope 19. A signal of the captured image output from an image sensor 35 (see Fig. 5) provided inside the rigid portion 25 is transmitted from the plug portion 21 to the video processor 13 via the transmission cable 33.

The video processor 13 performs various image processes (see above) on the signal of the captured image transmitted via the transmission cable 33, converts data of the captured image after the image processes into a display signal, and outputs the signal to the monitor 17.

The monitor 17 is composed of a display device such as a Liquid Crystal Display (LCD) or a Cathode Ray Tube (CRT). The monitor 17 displays a captured image of an observation target captured by the oblique-viewing endoscope 15. The monitor 17 displays a visible light image captured under, for example, visible light (that is, white light) illumination for illuminating an observation target, which is guided from, for example, the video processor 13 to the rigid portion 25 via the plug portion 21.

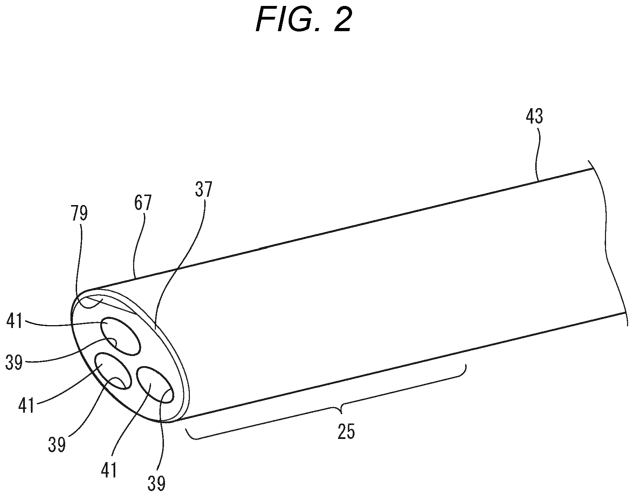

Fig. 2 is a perspective view of the rigid portion 25 illustrated in Fig. 1. In the oblique-viewing endoscope 15, the rigid portion 25 has an endoscope tip component 37. The endoscope tip component 37 can be formed of a rigid metal (for example, stainless steel) or a resin formed so as to have rigidity. The endoscope tip component 37 is formed, for example, in the shape of an elliptical plate. When an endoscope forming the endoscope system 11 is a direct endoscope, the endoscope tip component 37 may be formed in a disk shape. As illustrated in Fig. 2, a plurality of window through holes 39 are bored in the endoscope tip component 37. In the present embodiment, the three window through holes 39 are arranged in a radial direction from a center of the endoscope tip component 37. Each window through hole 39 is airtightly blocked by adhesively fixing a cover glass 41 for objectives. In the oblique-viewing endoscope 15, the endoscope tip component 37 and a tip portion of a sheath 43 adhesively fixed to the endoscope tip component 37 form the rigid portion 25 having a columnar appearance. In the oblique-viewing endoscope 15 according to the present embodiment, a length of the rigid portion 25 is not particularly limited to a length illustrated in Fig. 2 as long as the oblique-viewing endoscope 15 satisfies the ease of insertion in a subject.

Fig. 3 is a perspective view as seen through an inside of the rigid portion 25 illustrated in Fig. 2. The oblique-viewing endoscope 15 has a 3D camera module 45 as an example of an endoscope module inside the rigid portion 25. The 3D sub-frame (hereinafter referred to as "3D camera module 45") is composed of a plurality of imaging modules 47. In the present embodiment, the 3D camera module 45 is composed of, for example, two imaging modules 47 in order to capture an image forming a three-dimensional image.

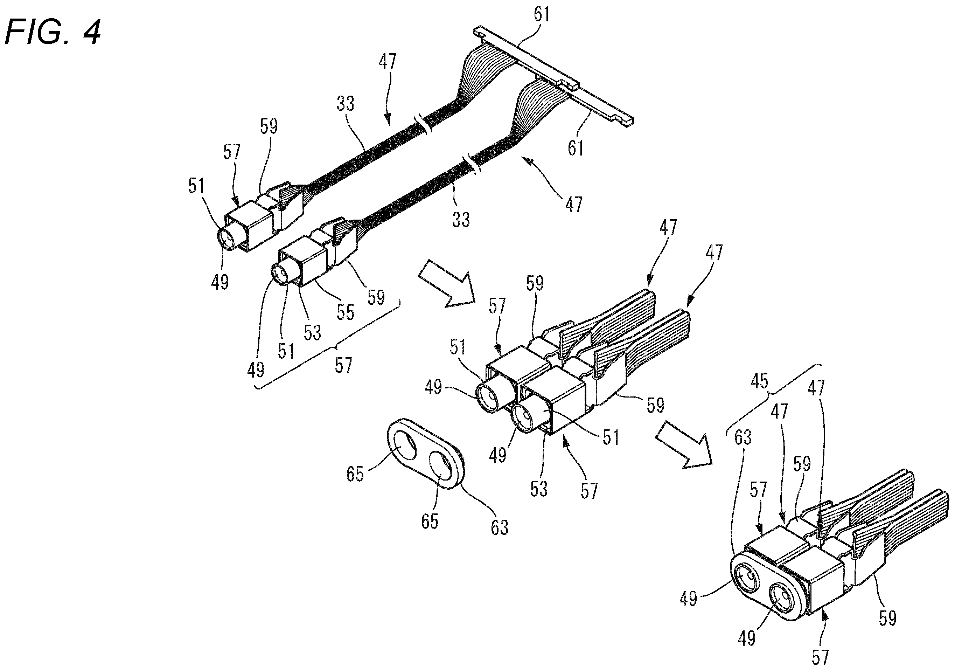

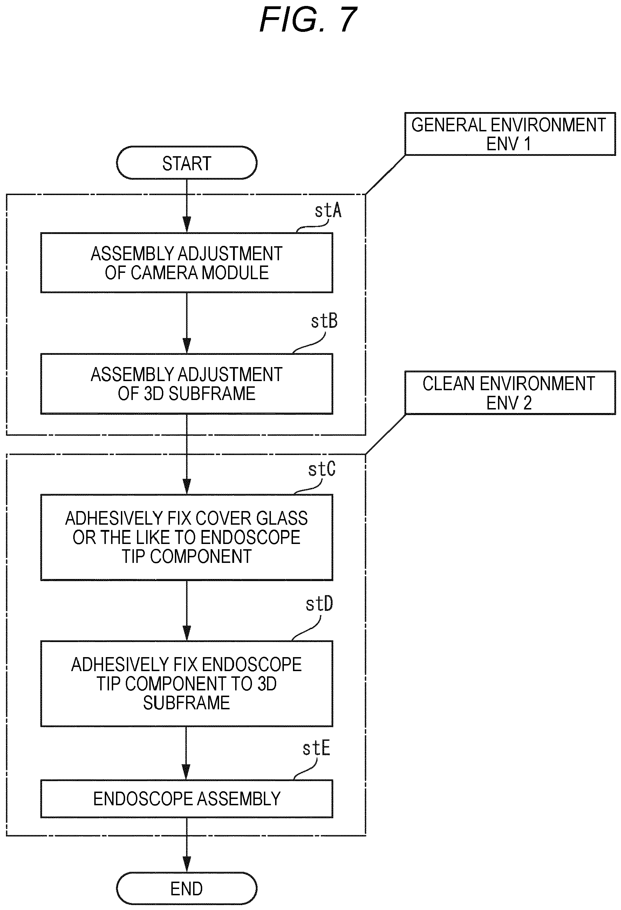

Fig. 4 is an explanatory diagram illustrating an example of an assembly procedure of the 3D camera module 45. In the respective imaging modules 47 which form the 3D camera module 45, a lens barrel 51 containing a lens 49, which is an optical system, an image sensor 35 (see Fig. 5), and a sensor holding member 53 that relatively fixes the lens barrel 51 and the image sensor 35 are assembled in a first atmosphere ENV1 (see Fig. 7). Here, the first atmosphere is, for example, an environment (in other words, a general assembly work environment) which is clean enough to assemble precision electronic components. An outside of the sensor holding member 53 is further covered with a square tubular cover 55. The lens 49, the lens barrel 51, the image sensor 35, the sensor holding member 53, the cover 55, and a flexible substrate 59 (see below) form a camera unit 57.

The camera unit 57 has a plurality of pads (not illustrated) on a back surface of the image sensor 35. The flexible substrate 59 is electrically connected to the back surface of the image sensor 35 via the pad. The flexible substrate 59 is disposed between the image sensor 35 and the transmission cable 33. For the transmission cable 33, for example, a flat cable in which a plurality of parallel insulating conductors are formed in a strip on the same plane is used. A transmission circuit in which a plurality of linear conductors are patterned and printed is formed on the flexible substrate 59. The flexible substrate 59 conductively connects each electric wire of the transmission cable 33 to the transmission circuit. As a result, the image sensor 35 is connected to the transmission cable 33 via the flexible substrate 59. As the flexible substrate 59, a Flexible Flat Cable (FFC), which is formed into a flexible strip cable by covering a conductor composed of a plurality of strip-shaped thin plates with an insulating sheet material, a Flexible Printed Circuit board (FPC) in which a linear conductor is pattern-printed on a flexible insulating substrate, or the like can be used.

A connector 61 is connected to a base end of the transmission cable 33. The connector 61 is accommodated in the plug portion 21 (see Fig. 1) described above and can be electrically connected to the socket portion 31. The camera unit 57, the transmission cable 33, and the connector 61 form one imaging module 47.

The 3D camera module 45 includes two imaging modules 47 and a sub-frame 63 that relatively fixes each of the two imaging modules 47. The sub-frame 63 and the two imaging modules 47 are accommodated in an outer shell of a body of the oblique-viewing endoscope 15, which is assembled in a second atmosphere (more specifically, the second atmosphere, which is a cleaner environment than the first atmosphere) different from the first atmosphere. Here, the second atmosphere is an assembly work environment set to a higher cleanliness than the first atmosphere. A work of accommodating the 3D camera module 45 in the outer shell in the second atmosphere is carried out, for example, in a limited clean process with official approval.

The sub-frame 63 has a pair of lens barrel insertion holes 65 into which two lens barrels 51 are loosely fitted, respectively. In each of the pair of imaging modules 47, outer circumferences of the two lens barrels 51 positioned with each other are adhesively fixed to inner circumferences of the lens barrel insertion holes 65.

The 3D camera module 45 can capture an image in which at least two of the two or more imaging modules 47 form the three-dimensional image.

Fig. 5 is a side cross-sectional view of the rigid portion 25 illustrated in Fig. 2. An outer shell 67 as an example of an outer shell portion of the oblique-viewing endoscope 15 includes the endoscope tip component 37 to which the sub-frame 63 is fixed and the sheath 43 in which a tubular tip opening 69 is blocked by the endoscope tip component 37. The plug portion 21 (see Fig. 1) is attached to a rear end of the sheath 43. The plug portion 21 accommodates the above-described connector 61 conductively connected to a base end of the transmission cable 33.

The sheath 43 is made of a flexible material and covers a part of the rigid portion 25 of the oblique-viewing endoscope 15 and an outer circumference of the soft portion 23. The tip opening 69 of the sheath 43 is inclined by a predetermined angle θ with respect to a virtual surface 73 perpendicular to an axis 71 of the rigid portion 25 to open. The endoscope tip component 37 is inclined with respect to the virtual surface 73 to block the tip opening 69. In the endoscope tip component 37, a chamfered portion 79 having an edge removed is formed on a forward inclined tip portion 77 on an inclined tip surface 75 (see Fig. 2). The sub-frame 63 and the endoscope tip component 37 are adhesively fixed by an adhesive agent 81.

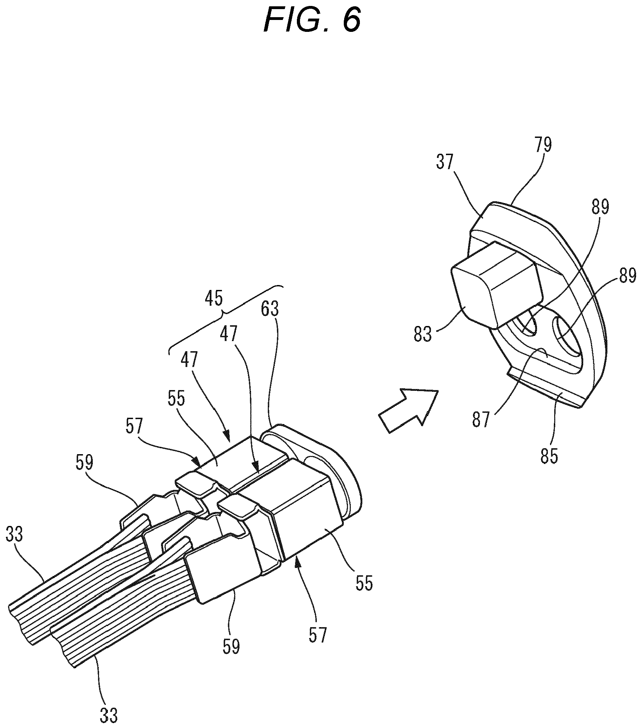

Fig. 6 is an exploded perspective view of the endoscope tip component 37 and the imaging module 47 illustrated in Fig. 3. A plurality of window through holes 39 are bored in the endoscope tip component 37 (see Fig. 2). In the present embodiment, three window through holes 39 corresponding to two imaging modules 47 and one light source 83 are bored. Each window through hole 39 is airtightly blocked by adhesively fixing the cover glass 41 for objectives. On a lower back surface of the endoscope tip component 37, a sheath fixing portion 85 projects along the axis 71 of the rigid portion 25. An inner circumference of the sheath 43 is fixed to the outer circumference of the sheath fixing portion 85.

The endoscope tip component 37 has a sub-frame fixing recess 87 that relatively positions and fixes the sub-frame 63. The sub-frame fixing recess 87 is an oval recess which is long in a parallel direction of the two imaging modules 47. At a bottom of the sub-frame fixing recess 87, lens barrel insertion holes 89 for individually inserting the lens barrels 51 of the two imaging modules 47 are bored. That is, as illustrated in Fig. 5, at the same time that the sub-frame 63 is adhesively fixed to the sub-frame fixing recess 87, the lens barrel 51, which is fixed to the lens barrel insertion hole 65 of the sub-frame 63 and protrudes, is adhesively fixed to the lens barrel insertion hole 89 of the endoscope tip component 37. The cover glass 41 is arranged in front of the lens barrel 51.

In the oblique-viewing endoscope 15, the light source 83 which irradiates the outside of the outer shell 67 with illumination light rays is disposed on a back surface of at least one cover glass 41. As the light source 83, for example, a Light Emission Diode (LED) as an example of a point light source can be preferably used. The LED is adhesively fixed to the endoscope tip component 37 so that a light emitting portion coincides with a back portion of one of the three window through holes 39. The light source 83 may incorporate not only the LED but also a cooling mechanism (not illustrated) as a heat dissipation measure during irradiation of light rays from the LED.

The oblique-viewing endoscope 15 having the above configuration can be said to have the following structure from a structural point of view. That is, the structure of the oblique-viewing endoscope 15 includes two or more imaging modules 47 each of which has the lens barrel 51 containing an optical system, the image sensor 35, and the sensor holding member 53 that relatively fixes the lens barrel 51 and the image sensor 35, and the sub-frame 63 for relatively fixing each of two or more imaging modules 47 is provided in the outer shell 67 of the body of the oblique-viewing endoscope 15.

Then, in the structure of the oblique-viewing endoscope 15, the sub-frame 63 is isolated from the outside of the outer shell 67. Here, isolation means that the sub-frame 63 is completely covered in a state where even a part of the sub-frame 63 does not appear outside the outer shell 67.

Next, a method of manufacturing the oblique-viewing endoscope 15 according to the present embodiment will be described.

Fig. 7 is a flowchart illustrating an example of a procedure of a method for manufacturing an oblique-viewing endoscope 15 according to the first embodiment. The oblique-viewing endoscope 15 is manufactured separately in the first atmosphere, which is a general environment, and in the second atmosphere, which is a cleaner environment than the general environment.

In Fig. 7, in the general environment, assembly adjustment (step stA illustrated in Fig. 7) of the imaging module 47 and assembly adjustment (step stB) of the 3D camera module 45 are performed. That is, in the first atmosphere ENV1, a process (step stA) of assembling the imaging module 47 by relatively fixing the lens barrel 51 containing the optical system and the image sensor 35 using the sensor holding member 53 and a process (step stB) of relatively fixing each of two or more imaging modules 47 using the sub-frame 63 are included.

On the other hand, in the clean environment, adhesive fixing (step stC) of the cover glass 41 or the like to the endoscope tip component 37, adhesive fixing (step stD) between the sub-frame 63 and the endoscope tip component 37, and endoscope assembly (step stE) in which the sheath 43 is fixed to the endoscope tip component 37 to which the sub-frame 63 is fixed are performed. That is, in the second atmosphere ENV2, which is cleaner than the first atmosphere ENV1, it includes a process (step stC) of adhesively fixing the cover glass 41 to the endoscope tip component 37, a process (step stD) of fixing the sub-frame 63 to the back surface of the endoscope tip component 37, and a process (step stE) of airtightly sealing the sub-frame 63 and two or more imaging modules 47 in the outer shell at the same time as forming the outer shell 67 of the endoscope body by fixing the endoscope tip component 37 to the tip opening 69 in the tubular sheath 43.

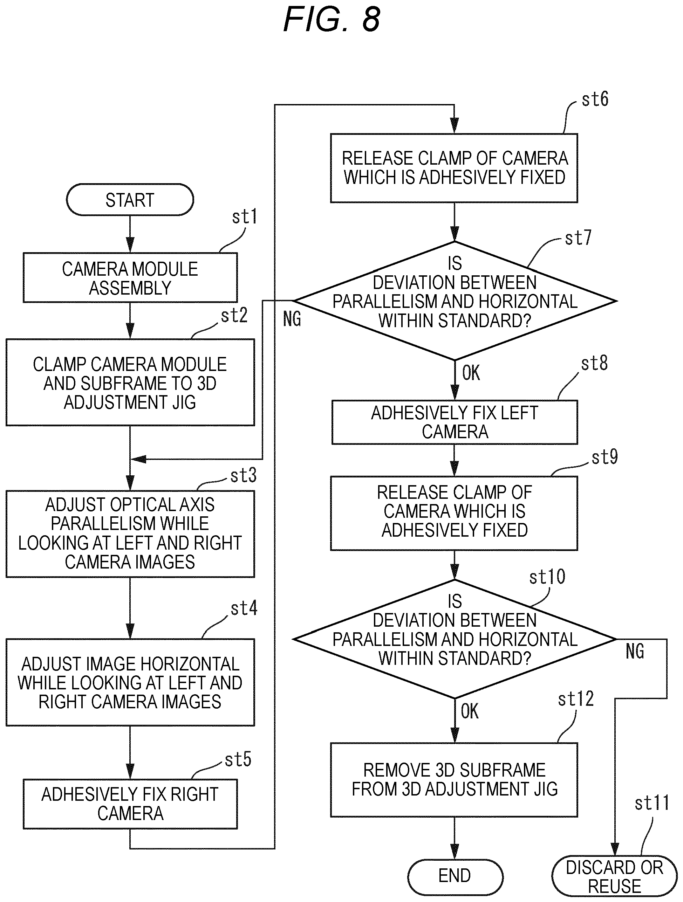

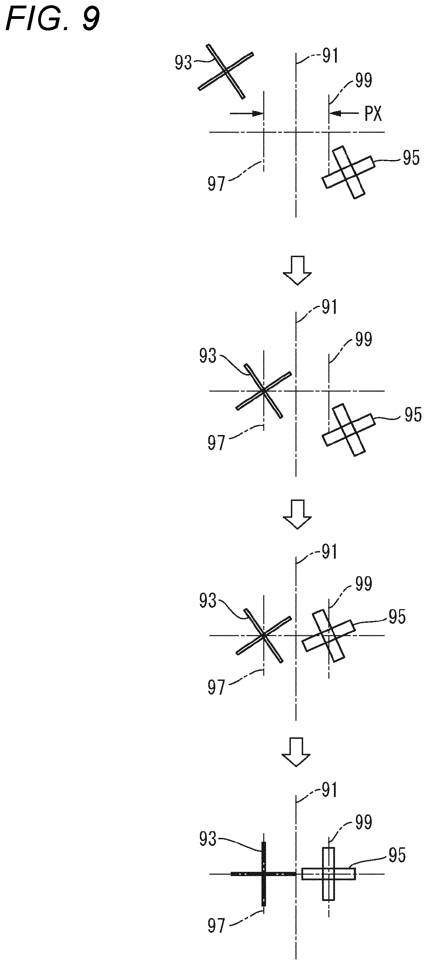

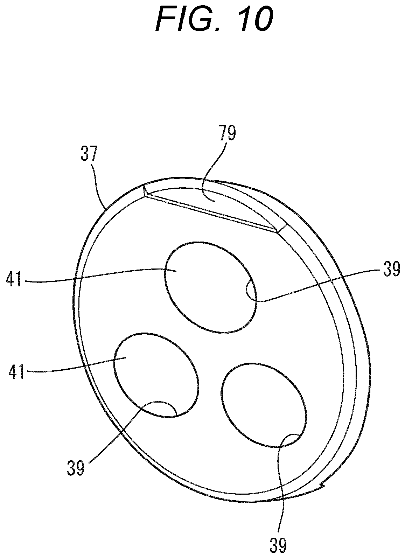

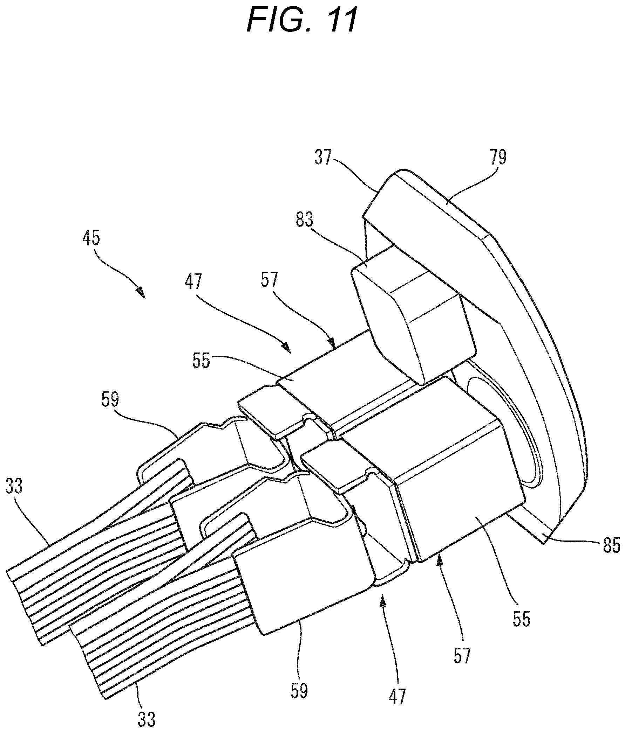

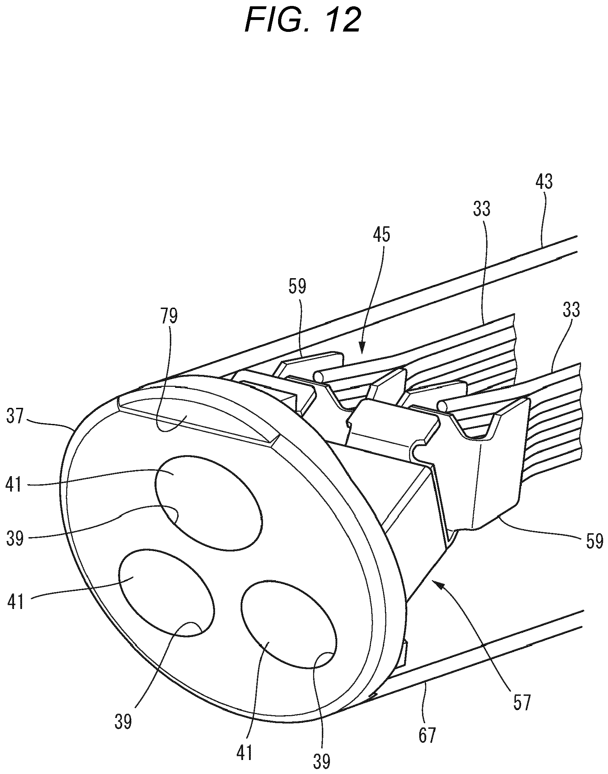

Fig. 8 is a flowchart illustrating an example of a procedure for adjusting the 3D sub-frame. Fig. 9 is a schematic view illustrating a procedure of optical axis parallelism adjustment and image horizontal adjustment of the 3D camera module 45. Fig. 10 is a perspective view of the endoscope tip component 37 to which the cover glass 41 is adhesively fixed. Fig. 11 is a perspective view of the 3D camera module 45 to which the endoscope tip component 37 is adhesively fixed. Fig. 12 is a perspective view as seen through a part of the rigid portion 25 in which the outer shell 67 is formed by the endoscope tip component 37 and the sheath 43. In the explanation of Fig. 8, Figs. 9 to 12 will be referred to as necessary.

In Fig. 8, in the assembly adjustment (step stA) of the imaging module 47, to start assembling the 3D camera module 45 (st1, see Fig. 8), first, the two imaging modules 47 and the sub-frame 63 are clamped (fixed) to a 3D adjustment jig (not illustrated) (st2).

In Fig. 9, a captured image of a target 91 is acquired from each of the two imaging modules 47 (that is, the left camera and the right camera) clamped to the 3D adjustment jig. In Fig. 9, the image of the target 91 is indicated by an alternate long and short dash line cross mark. A left camera image 93 illustrating the direction and rotation state of an optical axis in the left camera is indicated by a fine cross mark. A right camera image 95 illustrating the direction and rotation state of the optical axis in the right camera is indicated by a thick cross mark.

In the adjustment procedure, first, an operator adjusts the optical axis parallelism while looking at the left and right camera images (st3). For example, the optical axis of the left camera image 93 is aligned with a left scale 97 of the target 91. Next, the optical axis of the right camera image 95 is aligned with a right scale 99 of the target 91. The left scale 97 and the right scale 99 are set at equal distances to the left and right in a horizontal direction from a center of the target. The distance between the left and right scales is a parallax px.

Next, an operator performs the image horizontal adjustment while looking at the left and right camera images (st4). The image horizontal adjustment is performed by rotating each of the left and right cameras around the optical axis. After the optical axis parallelism adjustment and the image horizontal adjustment are completed, for example, the right camera (that is, one imaging module 47) is adhesively fixed to the sub-frame 63 by an operator (st5).

Next, an operator releases the clamp of the right camera which is adhesively fixed (st6). In a state where the right camera clamp is released, it is determined again whether the deviation between the optical axis parallelism and the image horizontal is within a standard (st7). When the deviation is no longer within the standard due to the release of the clamp, the adjustment is performed again from the optical axis parallelism adjustment. When the deviation is still within the standard even after releasing the clamp, the left camera (that is, the other imaging module 47) is adhesively fixed to the sub-frame 63 (st8).

Next, an operator releases the clamp of the left camera which is adhesively fixed (st9). Again, it is determined whether the deviation between the optical axis parallelism and the image horizontal is within the standard (st10). When the deviation is no longer within the standard due to the release of the clamp, the workpieces (that is, right and left cameras at work) are discarded or reused (st11). When the deviation is still within the standard even after the clamp is released, the completed workpiece is removed from the 3D adjustment jig (st12) and the assembly adjustment of the 3D camera module 45 in the first atmosphere, which is a general environment, is completed (stB).

On the other hand, in the second atmosphere, which is a clean environment, the cover glass 41 or the like is adhesively fixed to the window through hole 39 of the endoscope tip component 37 by the operator (stC).

Next, the operator adheres and fixes the endoscope tip component 37 to the sub-frame 63 of the 3D camera module 45 (stD). The endoscope tip component 37 is adhesively fixed in a state where the outer circumference of the 3D camera module 45 is inserted into the sub-frame fixing recess 87 (see Fig. 6). When the 3D camera module 45 is brought into the second atmosphere ENV2, the 3D camera module 45 may be sterilized if necessary.

Finally, the operator performs endoscope assembly in which the sheath 43 is fixed to the endoscope tip component 37 to which the sub-frame 63 is fixed (stE). In the endoscope assembly, the sheath 43 with the transmission cable 33 inside is sent to the endoscope tip component 37 and the tip opening 69 of the sheath 43 is joined to the outer circumference of the endoscope tip component 37. As a result, the sealing of the rigid portion 25 in the second atmosphere is completed.

A connector 61 is connected to the transmission cable 33 derived from a base end of the sheath 43. The plug portion 21 accommodating the connector 61 is attached to the base end of the sheath 43. This completes the production of the oblique-viewing endoscope 15.

Next, the operation of the oblique-viewing endoscope 15 according to the first embodiment will be described.

The endoscope module (3D camera module 45) according to the first embodiment includes two or more imaging modules 47 in each of which the lens barrel 51 containing the optical system, the image sensor 35, and the sensor holding member 53 that relatively fixes the lens barrel 51 and the image sensor 35 are assembled in the first atmosphere ENV1 and the sub-frame 63 which is assembled in the first atmosphere ENV1 and relatively fixes each of the two or more imaging modules 47. The sub-frame 63 and two or more imaging modules 47 are accommodated and fixed in the outer shell 67 of the endoscope body (that is, the body of the oblique-viewing endoscope 15) assembled in the second atmosphere ENV2, which is cleaner than the first atmosphere ENV1.

In the 3D camera module 45 according to the first embodiment, two or more imaging modules 47 are accommodated and fixed in the outer shell of the endoscope body. Each imaging module 47 is assembled by the lens barrel 51 containing an optical system, the image sensor 35, and the sensor holding member 53 that relatively fixes the lens barrel 51 and the image sensor 35. In the imaging module 47, the optical system and the image sensor 35 are relatively aligned by the sensor holding member 53, so that the imaging light ray from the subject is optimally imaged in a light receiving region of the image sensor 35. Each of the imaging modules 47 assembled in this way is assembled by the sub-frame 63 in the first atmosphere ENV1 so as to be relatively positioned and integrated.

The two or more imaging modules 47 integrated via the sub-frame 63 are accommodated in the outer shell of the endoscope body assembled in the second atmosphere ENV2, which is cleaner than the first atmosphere ENV1. That is, the endoscope accommodates and seals the imaging module 47 in which two or more are integrated in the outer shell of the endoscope body.

Here, the imaging module 47 in which the lens barrel 51 and the image sensor 35 are fixed using the sensor holding member 53 is assembled using a precise adjustment jig or the like. The two or more imaging modules 47 are relatively positioned and integrated by using a precise adjustment jig or the like. Assembling the imaging module 47 as a single unit and assembling two or more imaging modules 47 together by the sub-frame 63 are performed in the same first atmosphere. Since each member assembled in the first atmospheres does not come into contact with a patient, it is possible to work in an atmosphere in which the cleanliness is relatively relaxed.

On the other hand, the imaging module 47, which is assembled in the first atmosphere and two or more are integrated by the sub-frame 63, is accommodated in the outer shell in the second atmosphere. The second atmosphere is set to a higher degree of cleanliness than the first atmosphere. The work of accommodating two or more integrated imaging modules 47 in the outer shell in the second atmosphere is carried out in a clean process with official approval. In other words, in the 3D camera module 45, the process which requires cleanliness remarkably can be completed with the minimum work of only accommodating the imaging module 47 in which two or more are integrated in the outer shell.

In this way, since the 3D camera module 45 is provided with the sub-frame 63 for relatively positioning and fixing two or more imaging modules 47, it can be assembled using a precision adjustment jig and the like in the first atmosphere where the cleanliness is relatively relaxed. Accordingly, it is not necessary to install and assemble the precision adjustment jigs and the like in the second atmosphere with high cleanliness.

As a result, in the 3D camera module 45, the internal manufacturing process that does not include the patient contact portion and the external manufacturing process that includes the patient contact portion can be separated, so that it is not necessary to maintain remarkable cleanliness in the internal manufacturing process. Therefore, process management and process construction costs can be reduced. Since the camera unit assembly process, which requires a precise adjustment jig and the like particularly, can be separated, it is possible to reduce the process construction cost significantly.

The 3D camera module 45 captures an image in which at least two of the two or more imaging modules 47 form the three-dimensional image.

In the 3D camera module 45, at least two of the two or more imaging modules 47 become the imaging modules 47 for capturing an image (for example, a left image and a right image) forming the three-dimensional image. The two imaging modules 47 for capturing images forming the three-dimensional image have the same specifications and the optical axes of the two imaging modules 47 are parallel to each other. Therefore, the optical axes of the two imaging modules 47 are separated by a certain distance. Under such imaging conditions, the 3D camera module 45 ensures that the imaging surfaces of the two imaging modules 47 are flush with each other. In the two imaging modules 47, the deviation of the coordinates of the images at the same point in the same subject of the two captured images, each of which has coordinates, is the parallax px.

In the 3D camera module 45, a plurality of different captured images can be imageprocessed by the parallax px acquired from the plurality of interlocking imaging modules 47 and a stereoscopic image reflecting the depth information can be displayed on the display device.

In the 3D camera module 45, the sub-frame 63 has a pair of lens barrel insertion holes 65 in which each of the two lens barrels 51 are loosely fitted and the outer circumferences of the two lens barrels 51 positioned with each other are adhesively fixed to the inner circumferences of the lens barrel insertion holes 65.

In the 3D camera module 45, the sub-frame 63 has a pair of lens barrel insertion holes 65 that fit (loosely fit) each of the two lens barrels 51 in a loosely fitted state. That is, there is a gap between the lens barrel 51 and the lens barrel insertion hole 65 within which the lens barrel 51 can move.

As a result, the two lens barrels 51 can move in a state of being inserted into the lens barrel insertion holes 65 and rotation adjustment around the optical axis of the optical system and swing adjustment around the axis orthogonal to the optical axis can be performed in a state where the lens barrel 51 is inserted into the lens barrel insertion hole 65. In the two lens barrels 51, which are positioned with each other and positioned with respect to the sub-frame 63, and the sub-frame 63, the outer circumference of the lens barrel 51 can be adhesively fixed to the inner circumference of the lens barrel insertion hole 65 by the adhesive agent 81. The adhesive strength and position accuracy can be maintained for a long period of time by filling a space around a side surface of the lens barrel 51 extending from the opening of the lens barrel insertion hole 65 to the sensor holding member 53 with the adhesive agent 81 and then curing.

As a result, the two lens barrels 51 are positioned with respect to the sub-frame 63. Therefore, in the 3D camera module 45, when the sub-frame 63 is positioned in a predetermined position with respect to the outer shell 67, the optical systems of the two imaging modules 47 are positioned simultaneously with respect to the outer shell 67 (rigid portion 25).

The oblique-viewing endoscope 15 according to the first embodiment includes two or more imaging modules 47 in each of which the lens barrel 51 containing the optical system, the image sensor 35, and the sensor holding member 53 that relatively fixes the lens barrel 51 and the image sensor 35 are assembled in the first atmosphere ENV1, the sub-frame 63 which is assembled in the first atmosphere ENV1 and relatively fixes each of the two or more imaging modules 47, and the outer shell portion (for example, outer shell 67) that accommodates and fixes the sub-frame 63 and the two or more imaging modules 47 and is assembled in the second atmosphere ENV2 which is cleaner than the first atmosphere ENV1. The outer shell 67 has the endoscope tip component 37 to which the sub-frame 63 is fixed and the sheath 43 in which the tubular tip opening 69 is blocked by the endoscope tip component 37.

In the endoscope according to the first embodiment, the outer shell 67 of the endoscope body includes the endoscope tip component 37 and the sheath 43. The endoscope tip component 37 is formed in a disk shape or an elliptical plate shape. The endoscope tip component 37 is formed of a metal such as stainless steel. The inner peripheral surface of the sheath 43 is adhesively fixed to the outer peripheral surface of the endoscope tip component 37 in the thickness direction. In the endoscope tip component 37 in which the sheath 43 is fixed to the outer peripheral surface, an overhanging portion having an outer diameter larger than that of the outer peripheral surface by the thickness of the sheath 43 is formed at the tip. Therefore, in the endoscope tip component 37, the outer diameter of the overhanging portion and the outer diameter of the sheath 43 are flush with each other.

As a result, in the endoscope, by inserting the outer peripheral surface of the endoscope tip component 37 into the tip opening 69 of the sheath 43 and abutting the tip against the overhanging portion and then bonding and fixing the tip and the overhanging portion, a high-strength outer shell 67 can be assembled with a simple operation with the same outer diameter without steps.

The plug portion 21 that enables transmission and reception of power and various signals to and from the imaging module 47 via the transmission cable 33 inserted through the sheath 43 is connected to the rear end of the sheath 43.

As a result, the data of the captured image captured by the oblique-viewing endoscope 15 can be transmitted to the video processor 13, and thus a high-precision image captured by the oblique-viewing endoscope 15 can be displayed on the monitor 17 by a doctor or the like at the time of surgery or examination.

In the endoscope, the endoscope tip component 37 and the tip portion of the sheath 43 adhesively fixed to the endoscope tip component 37 form a columnar rigid portion 25. The tip opening 69 of the sheath 43 is inclined and opened with respect to the virtual surface 73 perpendicular to the axis 71 of the rigid portion 25 and the endoscope tip component 37 is inclined with respect to the virtual surface 73 to block the tip opening 69, and the chamfered portion 79 is formed at the forward inclined tip portion 77 in the inclined tip surface 75 of the endoscope tip component 37. When the endoscope is a forward-viewing endoscope, the tip opening 69 of the sheath 43 is opened without being inclined with respect to the virtual surface 73 perpendicular to the axis 71 of the rigid portion 25. The endoscope tip component 3 7 may block the tip opening 69 of the heath 43 and a chamfered portion (see, for example, chamfered portion 79) may be formed on the tip surface of the endoscope tip component 37.

In the endoscope, the tip opening 69 of the heath 43 is inclined and opened with respect to the virtual surface 73 perpendicular to the axis 71 of the rigid portion 25. The endoscope tip component 37 is inclined with respect to the virtual surface 73 to block the tip opening 69. That is, the endoscope is the oblique-viewing endoscope 15 in which the endoscope tip component 37 is inclined with respect to the virtual surface 73 perpendicular to the axis 71 of the rigid portion 25.

In the endoscope, the chamfered portion 79 is formed at the forward inclined tip portion 77, which is the foremost tip in the inclined tip surface 75 of the inclined endoscope tip component 37. The cylindrical rigid portion 25 has the sharp forward inclined tip portion 77 due to the inclination of the endoscope tip component 37. By chamfering the sharp forward inclined tip portion 77, damage to a tube wall when inserting the endoscope into a body cavity such as a blood vessel is prevented and it is possible to enhance safety. The safety improvement can be similarly applied by forming a chamfered portion (see, for example, chamfered portion 79) on the tip surface of the endoscope tip component 37 even when the endoscope is the forward-viewing endoscope.

In the endoscope, a plurality of window through holes 39 are bored in the endoscope tip component 37 and each window through hole 39 is airtightly sealed by the cover glass 41.

In the endoscope, a plurality of window through holes 39 are bored in the endoscope tip component 37. The window through hole 39 is sealed with the cover glass 41. Therefore, the endoscope tip component 37 is attached by blocking the tip opening 69 of the heath 43, so that the inside of the endoscope is airtightly shielded from the outside by the outer shell 67 composed of the endoscope tip component 37 and the heath 43.

Each of the cover glass 41 provided by blocking the plurality of window through holes 39 makes it possible to take in the imaging light ray from the outside and emit the illumination light ray from the inside. As a result, the endoscope seals the tip opening 69 of the sheath 43 with the endoscope tip component 37 that enables the entrance of the imaging light ray and the emission of the illumination light ray, in such a manner that the internal structure can be sealed at the same time while ensuring the light receiving function and the lighting function.

In the endoscope, the endoscope tip component 37 has the sub-frame fixing recess 87 that relatively fixes the sub-frame 63.

In the endoscope, the endoscope tip component 37 has the sub-frame fixing recess 87 that relatively fixes the sub-frame 63. By fixing the imaging module 47 to the sub-frame fixing recess 87, the imaging module 47 in which the two imaging modules 47 are positioned with each other via the sub-frame 63 is also positioned with respect to the endoscope tip component 37 via the sub-frame 63.

Therefore, the two imaging modules 47 are positioned and fixed to the endoscope tip component 37 via the sub-frame 63 and the sub-frame fixing recess 87 and can be simultaneously positioned for each window through hole 39 blocked by the cover glass 41.

In the endoscope, the light source 83 which irradiates the outside of the outer shell 67 with illumination light ray is disposed on the back surface of at least one cover glass 41.

In the endoscope, the light source 83 which irradiates the outside of the outer shell 67 with illumination light rays is disposed on the back surface of at least one cover glass 41. The light source 83 can be, for example, an LED adhesively fixed to the back surface of the endoscope tip component 37.

In the endoscope in which the light source 83 is directly attached to the endoscope tip component 37, compared to a light guide having a small radius of curvature and a large radiation loss, a sufficient amount of light can be obtained, and thus the subject can be illuminated sufficiently brightly.

In the endoscope, the light source 83 is a Light Emission Diode (LED) and the LED and the cooling mechanism of the LED may be disposed as the light source 83 on the back surface of the cover glass 41.

As a result, when the LED is used as the light source 83, measures to dissipate heat generated from the LED are taken. Therefore, it is possible to suppress an adverse effect due to heat propagation to the endoscope tip component 37.

In the endoscope, the light source 83 may be an optical fiber covered with the sheath 43 and extending from the base end side of the endoscope to the endoscope tip component 37.

As a result, it is possible to avoid using an LED at the tip, so that it is easier to reduce the size of the endoscope tip component 37 as compared with the case where the LED is used as a light source. Therefore, since light rays can be guided from the base end side of the endoscope to the endoscope tip component 37 by an optical fiber, it is possible to illuminate the subject sufficiently brightly as in the case of an LED.

The oblique-viewing endoscope 15 according to the first embodiment includes two or more imaging modules 47 having the image sensor 35, the lens barrel 51 containing an optical system, and the sensor holding member 53 that relatively fixes the lens barrel 51 and the image sensor 35, the sub-frame 63 that relatively fixes each of two or more imaging modules 47, and an outer shell portion (for example, the outer shell 67) that accommodates the sub-frame 63 and two or more imaging modules 47.

In the endoscope structure according to the first embodiment, two or more imaging modules 47 are accommodated in the outer shell of the endoscope body. Each imaging module 47 is assembled by the lens barrel 51 containing an optical system, the image sensor 35, and the sensor holding member 53 that relatively fixes the lens barrel 51 and the image sensor 35. In the imaging module 47, the optical system and the image sensor 35 are relatively aligned by the sensor holding member 53, so that the imaging light ray from the subject is optimally imaged in the light receiving region of the image sensor 35. Each of the imaging modules 47 assembled in this way is relatively positioned and integrated by the sub-frame 63.

Two or more imaging modules 47 integrated via the sub-frame 63 are accommodated in the outer shell of the endoscope body. That is, the endoscope structure can accommodate and seal the imaging module 47 in which two or more are integrated in the outer shell of the endoscope body.

In the oblique-viewing endoscope 15, the sub-frame 63 is isolated from the outside of the outer shell 67.

In the endoscopic structure, the sub-frame 63 is isolated from the outside of the outer shell 67. The imaging module 47 that fixes the lens barrel 51 and the image sensor 35 using the sensor holding member 53 is assembled using a precise adjustment jig or the like. The two or more imaging modules 47 are relatively positioned and integrated by using a precise adjustment jig or the like. Assembling the imaging module 47 as a single unit and assembling two or more imaging modules 47 together by the sub-frame 63 is an assembly of a part which does not come into contact with a patient. Therefore, it is possible to work in an atmosphere where the cleanliness is relatively relaxed.

On the other hand, the work of accommodating the integrated two or more imaging modules 47 in the outer shell is an assembly of a part which comes into contact with a patient, so it is necessary to be performed in a clean process with official approval.

Therefore, in the endoscopic structure, the process which requires cleanliness remarkably can be completed with the minimum work of only accommodating the imaging module 47 in which two or more are integrated in the outer shell.

In this way, since the oblique-viewing endoscope 15 is provided with the sub-frame 63 for relatively positioning and fixing two or more imaging modules 47, it can be assembled using a precision adjustment jig and the like in a manufacturing environment where the cleanliness is relatively relaxed. Accordingly, it is not necessary to install the precision adjustment jigs and the like in a highly clean manufacturing environment for assembly.

As a result, in the oblique-viewing endoscope 15, the internal manufacturing process which does not include the patient contact portion and the external manufacturing process which includes the patient contact portion can be separated, so that it is not necessary to maintain remarkable cleanliness in the internal manufacturing process. Therefore, process management and process construction costs can be reduced. Since the camera unit assembly process, which requires a precise adjustment jig and the like particularly, can be separated, it is possible to reduce the process construction cost significantly.

The method for manufacturing the endoscope according to the first embodiment includes, in the first atmosphere, a process of assembling the imaging module 47 by relatively fixing the lens barrel 51 containing the optical system and the image sensor 35 using the sensor holding member 53 and a process of relatively fixing each of two or more imaging modules 47 using the sub-frame 63. The method for manufacturing the endoscope according to the first embodiment includes, in the second atmosphere which is different from the first atmosphere, a process of fixing the sub-frame 63 to the back surface of the endoscope tip component 37 and a process of airtightly sealing the sub-frame 63 and two or more imaging modules 47 in the outer shell at the same time as forming the outer shell 67 of the endoscope body by fixing the endoscope tip component 37 to the tip opening 69 in the tubular sheath 43.

In the method for manufacturing the endoscope according to the first embodiment, the oblique-viewing endoscope 15 is assembled in two different atmospheres, in the first atmosphere ENV1 and the second atmosphere ENV2. The second atmosphere ENV2 is set to a higher degree of cleanliness than the first atmosphere ENV1.

In the first atmosphere, the lens barrel 51 and the image sensor 35 are fixed by using the sensor holding member 53 and the imaging module 47 is assembled. The assembly of the imaging module 47 is performed using a precision adjustment jig or the like. In the first atmosphere, two or more imaging modules 47 are relatively positioned by using a precise adjustment jig or the like and are integrally assembled via the sub-frame 63.

That is, assembling the imaging module 47 as a single unit and assembling two or more imaging modules 47 together by the sub-frame 63 are performed in the same first atmosphere. Since each member assembled in the first atmospheres does not come into contact with a patient, it is possible to work in an atmosphere in which the cleanliness is relatively relaxed.

On the other hand, the imaging module 47, which is assembled in the first atmosphere and two or more are integrated by the sub-frame 63, is accommodated in the outer shell in the second atmosphere ENV2. In other words, in the 3D camera module 45, the process which requires cleanliness remarkably is completed with the minimum work of only accommodating the imaging module 47 in which two or more are integrated in the outer shell.

In this way, in the method for manufacturing the endoscope, the sub-frame 63 for relatively positioning and fixing two or more imaging modules 47 is used, so that it can be assembled using a precision adjustment jig and the like in the first atmosphere where the cleanliness is relatively relaxed. Accordingly, it is not necessary to install and assemble the precision adjustment jigs and the like in the second atmosphere with high cleanliness.

As a result, in the method for manufacturing the endoscope, the internal manufacturing process which does not include the patient contact portion and the external manufacturing process which includes the patient contact portion can be separated, so that it is not necessary to maintain remarkable cleanliness in the internal manufacturing process. Therefore, process management and process construction costs can be reduced. Since the camera unit assembly process, which requires a precise adjustment jig and the like particularly, can be separated, it is possible to reduce the process construction cost significantly.

Therefore, according to the 3D camera module 45, the oblique-viewing endoscope 15, the endoscope structure, and the method for manufacturing the endoscope according to the first embodiment, it is possible to separate the assembly process which requires cleanliness from the assembly process which does not require cleanliness and it is possible to suppress an increase in manufacturing costs.

Although various embodiments are described above with reference to the drawings, it goes without saying that the present disclosure is not limited to such examples. It is clear that a person skilled in the art can come up with various modification examples, amendment examples, replacement examples, additional examples, deletion examples, and equal examples within the scope of claims and it is understood that they also belong to the technical scope of the present disclosure. Each component in the various embodiments described above may be arbitrarily combined as long as the gist of the invention is not deviated.

The present disclosure is useful as an endoscope module, an endoscope, and a method for manufacturing an endoscope which can separate an assembly process which requires cleanliness and an assembly process which does not require cleanliness and suppress an increase in manufacturing costs.

## Claims

1. An endoscope comprising:
two or more imaging modules having a lens barrel containing an optical system, an image sensor, and a sensor holding member that relatively fixes the lens barrel and the image sensor;
a sub-frame that relatively fixes each of the two or more imaging modules; and
an outer shell portion that accommodates and fixes the sub-frame and the two or more imaging modules.

2. The endoscope according to claim 1, wherein
the sub-frame is isolated from an outside of the outer shell portion.

3. The endoscope according to claim 1, wherein
the two or more imaging modules are assembled in a first working environment, and the sub-frame and the two or more imaging modules are assembled in an inside of the outer shell portion in a second working environment different from the first working environment.

4. The endoscope according to claim 1, wherein
at least two of the two or more imaging modules capture images which form a three-dimensional image.

5. The endoscope according to claim 4, wherein
the sub-frame has a pair of lens barrel insertion holes that loosely fit each of the two lens barrels, and an outer circumference of each of the two mutually positioned lens barrels is adhesively fixed to an inner circumference of the lens barrel insertion hole.
